# EUROPÄISCHE PATENTSCHRIFT

(11) **EP 0 575 909 B1**
(45) Veröffentlichungstag und Bekanntmachung des Hinweises auf die Patenterteilung: **17.09.1997**
(21) Anmeldenummer: 93109772.9
(22) Anmeldetag: 18.06.1993
(51) Int. Cl.: C09B 62/09

(54) **Wasserlösliche Disazoverbindungen, Verfahren zu ihrer Herstellung und ihre Verwendung als Farbstoffe**
Water-soluble disazo compounds, process for their preparation and their use as dyestuffs
Composés disazoiques solubles dans l'eau, procédé pour leur préparation et leur utilisation comme colorants

(30) Priorität: 25.06.1992 DE 4220834
(43) Veröffentlichungstag der Anmeldung: 29.12.1993
(73) Patentinhaber: HOECHST AKTIENGESELLSCHAFT, 65926 Frankfurt am Main (DE)
(72) Erfinder: von der Eltz, Andreas, Dr., W-6000 Frankfurt am Main 50 (DE); Russ, Werner Hubert, Dr., W-6093 Flörsheim am Main (DE)

(56) Entgegenhaltungen:
- EP-A- 0 319 845

## Beschreibung

Die Erfindung liegt auf dem technischen Gebiet der faserreaktiven Azofarbstoffe.

Aus der europäischen Patentanmeldungs-Veröffentlichung Nr. 0 042 108A, den US-Patentschriften Nrs. 4 798 887 und 5 679 347 und der britischen Patentschrift Nr. 1 102 204 sind Phenylazonaphthyl-azonaphthylamino-Verbindungen bekannt, an deren Aminogruppe ein faserreaktiver Chlortriazinyl-Rest gebunden ist. Diese bekannten Disazoverbindungen liefern Färbungen mit brauner Nuance; jedoch sind ihre Farbstoffeigenschaften noch verbesserungswürdig, so beispielsweise hinsichtlich ihrer Farbstärke und, soweit es die Farbstoffe der US-PS 5 079 347 angeht, hinsichtlich der Licht- und Hypochloritbleichechtheiten der Färbungen.

Mit der vorliegenden Erfindung wurde nunmehr mit der Auffindung der nachstehend definierten Disazoverbindungen entsprechend der allgemeinen Formel (1) 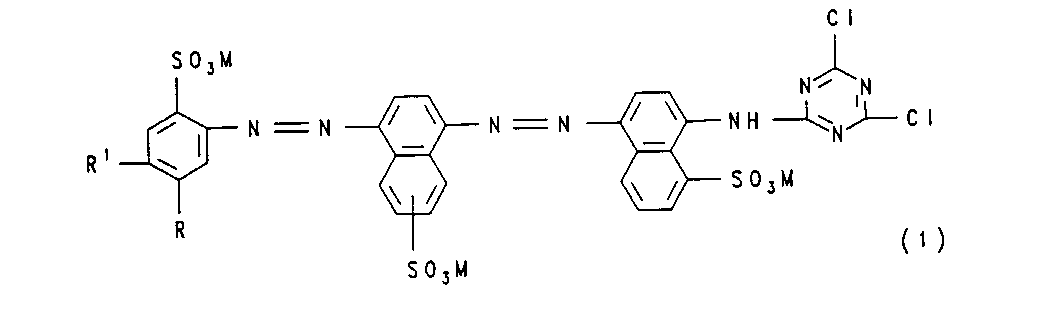 diese Aufgabe gelöst.

In dieser Formel (1) bedeuten:
- R: ist Wasserstoff oder Sulfo;
- R¹: ist Hydroxy, Methoxy oder Ethoxy, bevorzugt Methoxy;
- M: ist Wasserstoff oder ein Alkalimetall, wie Natrium, Kalium oder Lithium;
die freistehende Sulfogruppe in dem Naphthylenrest steht in 6- oder 7-Stellung.

Bevorzugt sind Disazoverbindungen der allgemeinen Formel (1), in welcher R¹ Methoxy bedeutet, des weiteren solche, in welcher R Wasserstoff ist.

In den vorstehenden wie in den nachstehenden Angaben bedeutet eine Sulfogruppe eine Gruppe der allgemeinen Formel -SO₃M mit M der obengenannten Bedeutung.

Die erfindungsgemäßen Disazoverbindungen können in Form ihrer freien Säure und bevorzugt in Form ihrer Salze, insbesondere neutralen Salze, wie der Alkalimetallsalze, vorliegen. Die Disazoverbindungen der allgemeinen Formel (1) finden bevorzugt in Form dieser Salze ihre Verwendung zum Färben und Bedrucken von hydroxy- und/oder carbonamidgruppenhaltigem Fasermaterial.

Die vorliegende Erfindung betrifft weiterhin ein Verfahren zur Herstellung der erfindungsgemäßen Disazoverbindungen, das dadurch gekennzeichnet ist, daß man eine Amino-Disazoverbindung der allgemeinen Formel (2) 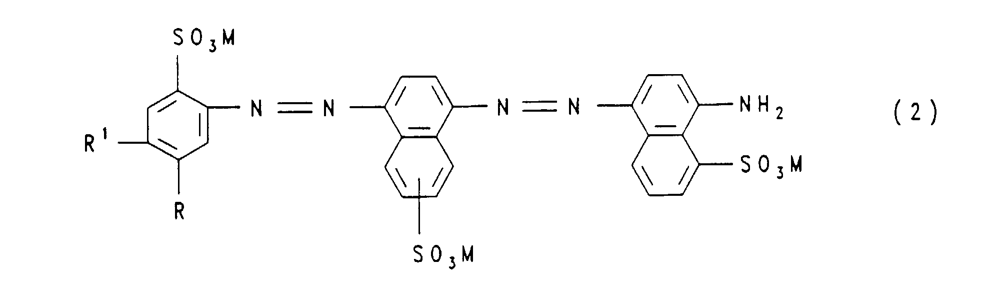 in welcher R, R¹ und M die obengenannten Bedeutungen besitzen, die in an und für sich üblicher und bekannter Weise durch Diazotierung und Kupplung der entsprechenden Komponenten hergestellt werden kann, mit Cyanurchlorid (2,4,6-Trichlor-1,3,5-triazin) umsetzt.

Die erfindungsgemäße Umsetzung einer Verbindung der allgemeinen Formel (2) mit Cyanurchlorid kann in wäßrig-organischem Medium (wobei der organische Lösemittelanteil bspw. Aceton, Dimethylformamid, Dioxan oder Dimethylsulfoxid ist) durchgeführt werden; bevorzugt erfolgt sie in wäßriger Lösung bzw. Suspension. Die Reaktionstemperatur liegt in der Regel zwischen -10°C und +30°C, bevorzugt zwischen +5°C und +25°C. In der Regel wird ein pH-Wert zwischen 4 und 7, bevorzugt zwischen 4 und 6,5, eingehalten.

Wie bereits erwähnt, erhält man die Ausgangsverbindungen der allgemeinen Formel (2) in üblicher Weise durch Diazotierung und Kupplungsreaktion. Diazokomponenten, die nach deren Diazotierung auf eine 6- oder 7-Sulfo-1 -aminonaphthalin-Verbindung gekuppelt werden, sind beispielsweise 2-Sulfo-4-methoxy-anilin, 2-Sulfo-4-ethoxy-anilin und 2,5-Disulfo-4-methoxy-anilin.

Die Abscheidung und Isolierung der erfindungsgemäß hergestellten Disazoverbindungen der allgemeinen Formel (1) aus den Syntheselösungen kann nach allgemein bekannten Methoden erfolgen, so beispielsweise entweder durch Ausfällen aus dem Reaktionsmedium mittels Elektrolyten, wie beispielsweise Natriumchlorid oder Kaliumchlorid, oder durch Eindampfen der Reaktionslösung, beispielsweise Sprühtrocknung, wobei der Syntheselösung eine Puffersubstanz zugefügt werden kann.

Die erfindungsgemäßen Disazoverbindungen der allgemeinen Formel (1) - im nachfolgenden Verbindungen (1) genannt - haben faserreaktive Farbstoffeigenschaften. Sie können deshalb zum Färben (einschließlich Bedrucken) von hydroxygruppenhaltigen und/oder carbonamigruppenhaltigen Fasermaterialien verwendet werden. Auch können die bei der Synthese der Verbindungen (1) anfallenden Lösungen, gegebenenfalls nach Zusatz einer Puffersubstanz und gegebenenfalls nach Konzentrierung, direkt als Flüssigpräparation der färberischen Verwendung zugeführt werden.

Gegenstand der vorliegenden Erfindung ist deshalb auch die Verwendung der erfindungsgemäßen Verbindungen (1) zum Färben (einschließlich Bedrucken) von hydroxy- und/oder carbonamidgruppenhaltigen Fasermaterialien bzw. Verfahren zu deren Anwendung auf diesen Substraten. Bevorzugt werden die Materialien in Form von Textilfasern, wie Garnen, Wickelkörpern und Geweben, eingesetzt. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten nach den für wasserlösliche Farbstoffe, insbesondere faserreaktive Farbstoffe, bekannten Anwendungstechniken applizieren und fixieren, so beispielsweise, indem man die Verbindung (1) in gelöster Form auf das Substrat aufbringt oder einbringt und sie durch Einwirkung von Wärme oder durch Einwirkung eines alkalisch wirkenden Mittels oder durch beide Maßnahmen auf bzw. in dem Material fixiert. Solche Färbe- und Fixierweisen sind sowohl in der Fachliteratur als auch in der Patentliteratur zahlreich beschrieben, wie beispielsweise in den Europäischen Patentanmeldungs-Veröffentlichungen Nrs. 0 078 009A und 0 181 585A.

Die Verbindungen (1) zeichnen sich durch eine hohe Farbstärke aus; mit ihnen werden deshalb sowohl auf carbonamidgruppenhaltigen Materialien, wie insbesondere auf Wolle, als auch auf hydroxygruppenhaltigem Material, wie insbesondere Cellulosefasermaterial, braune, insbesondere rotstichig braune, Färbungen und Drucke mit hoher Farbausbeute und gutem Farbaufbau bei sehr geringer Temperatur- und Alkaliabhängigkeit erhalten. Die Farbstoffe eignen sich zudem für die Trichromiefärbung. Die Färbungen und Drucke besitzen gute Echtheitseigenschaften, wie gute Licht- und Naßechtheitseigenschaften, so beispielsweise eine hohe Hypochloritbleichechtheit und hohe Naßlichtechtheiten des mit Trinkwasser oder einer sauren oder alkalischen Schweißlösung befeuchteten gefärbten Materials, des weiteren eine gute Plissierechtheit, Bügelechtheit und Reibechtheit, gute Waschechtheiten bei 60 bis 95°C, auch in Gegenwart von Perboraten, saure und alkalische Walk-, Überfärbe- und Schweißechtheiten, eine hohe Dämpfbeständigkeit, gute Alkali-, Säure-, Wasser- und Seewasserechtheiten und eine gute Säurelagerbeständigkeit ("acid fading") beim Lagern von feuchtem, säurehaltigem, gefärbtem Material (s. deutsche Auslegeschrift 2 322 236, Spalte 4, Zeilen 35 bis 42).

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter. Die in den Beispielen formelmäßig beschriebenen Verbindungen sind in Form der freien Säure angegeben; im allgemeinen werden sie in Form ihrer Alkalimetallsalze hergestellt und isoliert und in Form ihrer Salze zum Färben verwendet. Die im sichtbaren Bereich angegebenen Absorptionsmaxima (λₘₐₓ-Werte) wurden aus wäßriger Lösung der Alkalimetallsalze bestimmt.

### Beispiel 1

213 Teile 4-Methoxy-aminobenzol-3-sulfonsäure werden in 1000 Teilen einer schwefelsauren wäßrigen Lösung diazotiert; 223 Teile 1-Amino-naphthalin-6-sulfonsäure werden hinzugegeben, und die Kupplungsreaktion wird bei einem pH-Wert zwischen 4 und 5 und einer Temperatur zwischen 10 und 15°C durchgeführt. Die erhaltene Amino-Azoverbindung wird sodann in üblicher Weise diazotiert, überschüssige salpetrige Säure durch Amidosulfonsäure zerstört, und 223 Teile 1-Aminonaphthalin-8-sulfonsäure werden zu dem Ansatz gegeben, und die zweite Kupplungsreaktion wird gemäß den obigen Bedingungen durchgeführt. Die erhaltene Disazoverbindung wird durch Aussalzen mit Natriumchlorid isoliert und anschließend in eine feinverteilte Suspension von 194 Teilen Cyanurchlorid in 500 Teilen Eiswasser eingetragen. Die Umsetzung erfolgt bei einem pH-Wert von 6 und bei einer Temperatur zwischen 5 und 20°C. Nach Beendigung der Reaktion (kein Nachweis von freien Aminogruppen) werden zur Klärung der erhaltenen Syntheselösung 20 Teile Kieselgur hinzugegeben, und die Lösung wird anschließend filtriert.

Die erfindungsgemäße Disazoverbindung kann, gegebenenfalls unter Zugabe einer Puffersubstanz, durch Aussalzen mit Natriumchlorid, Filtration und Trocknen oder durch Sprühtrocknung isoliert werden. Man erhält eine elektrolytsalzhaltiges (vorwiegend natriumchloridhaltiges) Pulver des erfindungsgemäßen Alkalimetallsalzes (Natriumsalzes) der Verbindung der Formel

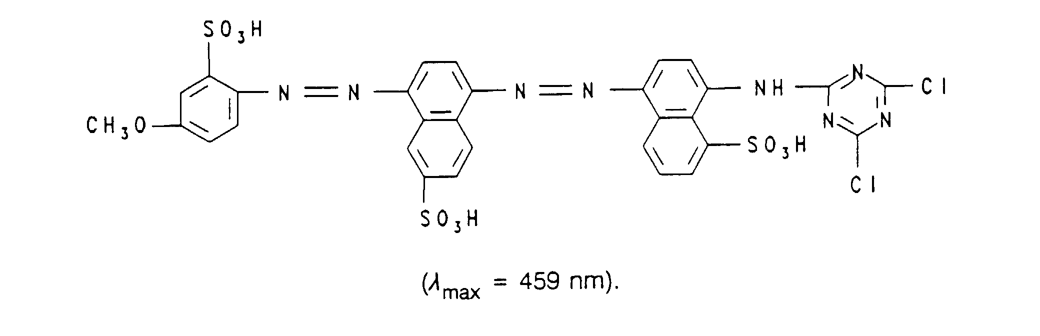

Diese Disazoverbindung besitzt sehr gute Farbstoffeigenschaften und liefert auf den in der Beschreibung genannten Materialien, insbesondere Cellulosefasermaterialien, wie Baumwolle, nach den in der Technik für Farbstoffe, insbesondere für faserreaktive Farbstoffe, üblichen Applikations- und Fixiermethoden und weitgehend unabhängig von der gewählten Temperatur und Alkalimenge farbstarke, rotstichig braune Färbungen und Drucke, die gute Echtheiten, insbesondere eine hohe Lichtechtheit und hohe Hypochloritechtheit, aufweisen.

### Beispiel 2

Zur Herstellung einer erfindungsgemäßen Disazoverbindung verfährt man gemäß der Verfahrensweise des Beispiels 1, setzt jedoch als Diazokomponente anstelle der 4-Methoxy-aminobenzol-3-sulfonsäure die äquimolare Menge an 4-Methoxy-aminobenzol-2,5-disulfonsäure ein. Man isoliert die erhaltene erfindungsgemäße Disazoverbindung der Formel (in Form der freien Säure geschrieben) 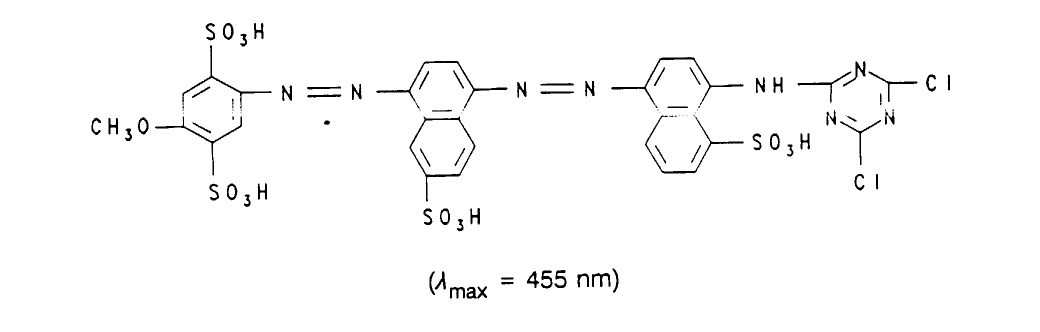 durch Aussalzen mit Natriumchlorid oder durch Sprühtrocknung als Natriumsalz. Die erfindungsgemäße Disazoverbindung besitzt ähnlich gute Farbstoffeigenschaften wie die des Beispiels 1.

## Patentansprüche

1. Eine Disazoverbindung entsprechend der allgemeinen Formel (1) 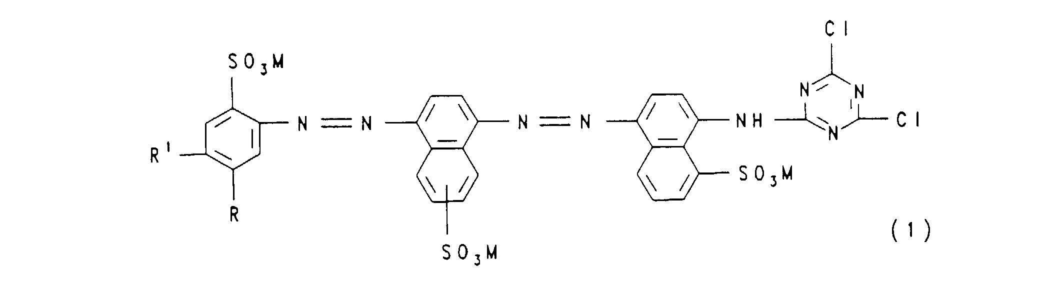 in welcher bedeuten:
R ist Wasserstoff oder Sulfo;
R¹ ist Hydroxy, Methoxy oder Ethoxy;
M ist Wasserstoff oder ein Alkalimetall;
die freistehende Sulfogruppe in dem Naphthylenrest steht in 6- oder 7-Stellung.

2. Disazoverbindung nach Anspruch 1, dadurch gekennzeichnet, daß R¹ Methoxy ist.

3. Disazoverbindung nach Anspruch 1 oder 2, dadurch gekennzeichnet, daß R Wasserstoff ist.

4. Verfahren zur Herstellung einer Disazoverbindung von Anspruch 1, dadurch gekennzeichnet, daß man eine Amino-Disazoverbindung der allgemeinen Formel (2) 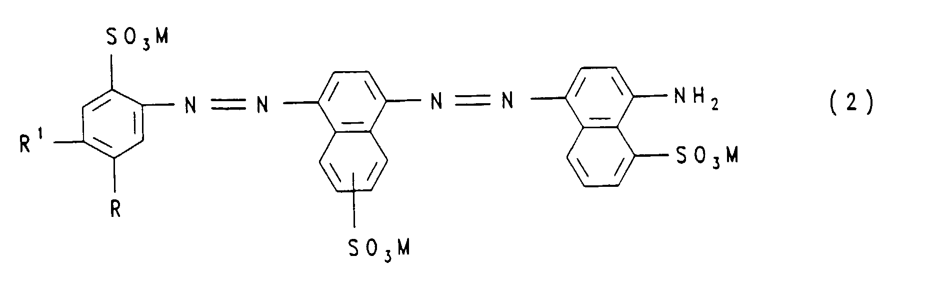 in welcher R, R¹ und M die in Anspruch 1 genannten Bedeutungen haben, mit Cyanurchlorid umsetzt.

5. Verwendung einer Verbindung der Formel (1) von Anspruch 1 zum Färben von hydroxy- und/oder carbonamidgruppenhaltigem Fasermaterial.

6. Verfahren zum Färben von hydroxy- und/oder carbonamidgruppenhaltigem Fasermaterial, bei welchem man einen Farbstoff auf das Material aufbringt oder einbringt und ihn mittels Wärme oder mittels einer säurebindenden Verbindung oder mittels beider Maßnahmen auf oder in dem Material fixiert, dadurch gekennzeichnet, daß man als Farbstoff eine Verbindung der Formel (1) von Anspruch 1 einsetzt.

## Claims

1. A disazo compound of the formula (1) 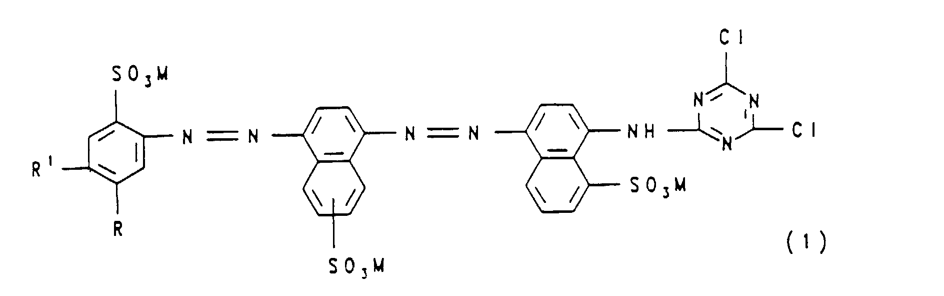 in which
R is hydrogen or sulfo;
R¹ is hydroxyl, methoxy or ethoxy;
M is hydrogen or an alkali metal;
the unassigned sulfo group in the naphthylene radical is in the 6 or 7 position.

2. A disazo compound as claimed in claim 1, wherein R¹ is methoxy.

3. A disazo compound as claimed in claim 1 or 2, wherein R is hydrogen.

4. A process for the preparation of a disazo compound as claimed in claim 1, which comprises reacting an aminodisazo compound of the formula (2) 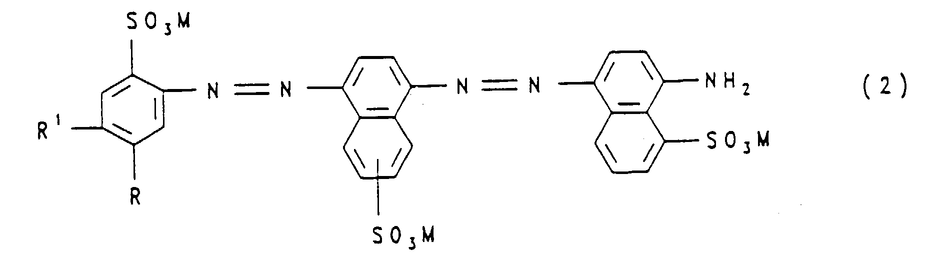 in which R, R¹ and M have the meanings given in claim 1, with cyanuric chloride.

5. Use of a compound of the formula (1) as claimed in claim 1 for the dyeing of hydroxyl- and/or carboxamido-containing fiber material.

6. A process for the dyeing of hydroxyl- and/or carboxamido-containing fiber material, in which a dye is applied to the material or incorporated therein and fixed on or in the material by means of heat or by means of an acid-binding compound or by means of both measures, which process comprises using a compound of the formula (1) of claim 1 as the dye.

## Revendications

1. Composé disazoique répondant à la formule générale (1) 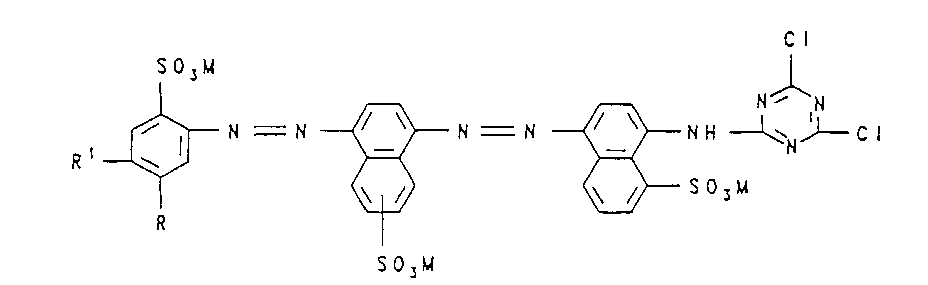 dans laquelle
R est un atome d'hydrogène ou sulfo ;
R¹ est hydroxy, méthoxy ou éthoxy ;
M est un atome d'hydrogène ou un métal alcalin ;
le groupe sulfo libre dans le radical naphtylène est en position 6 ou 7.

2. Composé disazoïque selon la revendication 1; caractérisé en ce que R¹ est méthoxy.

3. Composé disazoïque selon la revendication 1 ou 2, caractérisé en que R est l'hydrogène.

4. Procédé pour la préparation d'un composé disazoïque de la revendication 1, caractérisé en ce qu'on fait réagir avec le chlorure de cyanuryle un composé amino-disazoïque de formule générale (2) 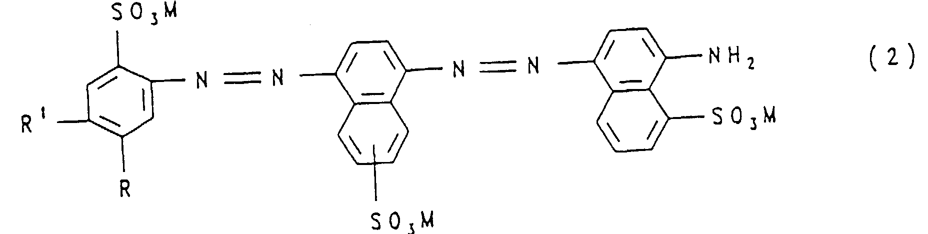 dans laqulle R, R¹ et M ont les significations données dans la revendication 1.

5. Utilisation d'un composé de formule (1) de la revendication 1, pour la teinture de matières fibreuses contenant des groupes hydroxy- et/ou carboxamido.

6. Procédé pour la teinture d'une matière fibreuse contenant des groupes hydroxy- et/ou carboxamido dans lequel on applique à la matière ou dans la matière introduit un colorant et on le fixe par la chaleur ou par un composé liant l'acide, ou à l'aide des deux mesures, à la matière ou dans la matière, caractérisé en ce qu'on utilise comme colorant un composé de formule (1) de la revendication 1.
